# EUROPEAN PATENT SPECIFICATION

(11) **EP 2 007 821 B1**
(45) Date of publication and mention of the grant of the patent: **12.05.2010**
(21) Application number: 07755615.7
(22) Date of filing: 17.04.2007
(51) Int. Cl.: C08F 10/00

(54) **SIMPLIFIED PROCESS TO PREPARE POLYOLEFINS FROM SATURATED HYDROCARBONS**
VEREINFACHTES VERFAHREN ZUR HERSTELLUNG VON POLYOLEFINEN AUS GESÄTTIGTEN KOHLENWASSERSTOFFEN
PROCEDE SIMPLIFIE DE PREPARATION DE POLYOLEFINES A PARTIR D'HYDROCARBURES SATURES

(30) Priority: 19.04.2006 US 406705
(43) Date of publication of application: 31.12.2008
(73) Proprietor: WESTLAKE LONGVIEW CORPORATION, Houston, TX 77056 (US)
(72) Inventor: VANDERBILT, Jeffrey James, Longview, Texas 75604-3637 (US); DEVON, Thomas James, Longview, Texas 75605-6169 (US); DOOLEY, Kenneth Alan, Longview, Texas 75604-4323 (US)
(74) Representative: Best, Michael
(86) International application number: PCT/US2007/009409
(87) International publication number: WO 2007/123918

(56) References cited:
- WO-A-00/48971
- GB-A- 823 932
- GB-A- 866 763
- US-A1- 2004 087 745
- "Kirk-Othmer Encyclopedia of Chemical technology" John Wiley and Sons, Inc. vol. 10, , pages 593-632
- "Technip Engineering and Technologies"
- Ravi Arora: "Separation of Ethane-Ethylene in High Flux Microchannels" Retrieved from the Internet: URL:http://aiche.confex.com/aiche/s08/tech program/P110164.HTM> [retrieved on 2009-05-15]

## Description

### FIELD OF THE INVENTION

The present invention generally relates to a process for preparing polyolefins from saturated hydrocarbons. The process features the use of crude olefin, unreacted alkane, and optionally crude oligomerized olefin.

### BACKGROUND OF THE INVENTION

Polyolefins, such as polyethylene, are typically prepared by polymerizing one or more olefins in the presence of a polymerization catalyst to form a polyolefin. Most commercially produced olefins, such as ethylene, are made by thermal cracking of hydrocarbons. Kirk-Othmer Encyclopedia of Chemical Technology, Vol. 9, p. 883 (4th ed. 1994). This process, however, suffers from a number of drawbacks such as low selectivity, high energy requirements, as well as multiple separation steps. *Id*. at 887-97.

Other methods for producing olefins are known, but few have been commercialized. One process that has been commercialized is the dehydrogenation of propane to propylene. But such approach must be run at high temperatures and is equilibrium-limited. *Id*. at 903.

Accordingly, there is a need for a simplified, more efficient process for preparing olefins and, in particular, for preparing polyolefins from alkanes.

### SUMMARY OF THE INVENTION

The present invention departs from the traditional ways of preparing polyolefins. It is a simplified process that does not involve separating unreacted alkanes from olefins before the polymerization step. Moreover, it involves the use of oxygen to reduce reaction temperatures and avoid equilibrium limitations.

Briefly, the present invention provides for a process for making polyolefin from an alkane. The process comprises:
(a) dehydrogenating an alkane in the presence of oxygen to form a dehydrogenation product stream comprising a corresponding alkene, unreacted alkane, and water, and optionally other by-products and oxygen;
(b) separating the water, other by-products (if present), and oxygen (if present) from the dehydrogenation product stream without separating the unreacted alkane to form a separated dehydrogenation product stream comprising alkene and unreacted alkane;
(c) polymerizing the alkene in the separated dehydrogenation product stream in the presence of a polymerization catalyst or initiator and the unreacted alkane to form a polymerization product stream comprising polyolefin, unreacted alkane, and optionally unreacted alkene;
(d) separating the polyolefin from the unreacted alkane and unreacted alkene (if present) in the polymerization product stream; and
(f) recycling of the unreacted alkane and unreacted alkene (if present) to the dehydrogenation step.

### DETAILED DESCRIPTION OF THE INVENTION

The process of the present invention is applicable to preparing a polymer from the corresponding alkane. Preferred alkanes include ethane and propane. The process can produce polyethylenes such as low density polyethylene (LDPE), linear low density polyethylene (LLDPE), and high density polyethylene (HDPE). The process can also produce polypropylene homopolymer, random copolymer, and block copolymer. Comonomers can be ethylene and/or higher α-olefins.

In the first step of the process according to the invention, an alkane is partially and selectively dehydrogenated in the presence of oxygen, to form a dehydrogenation product stream comprising the corresponding alkene, unreacted alkane, and water, and optionally other by-products (e.g., carbon dioxide and/or partial oxidation products) and oxygen.

Dehydrogenation can be carried out with oxygen in the presence of a catalyst over a wide range of temperatures, from 50 to greater than 600°C. In general, selectivity to alkene decreases and conversion to alkene and other by-products increases as temperature increases. A preferred temperature range is from about 100 to about 400°C. A more preferred temperature range is from about 100 to about 300°C. Pressure can be varied from atmospheric pressure to greater than 100 bar. Lower pressures are preferred.

All mentions herein of elements of Groups of the Periodic Table are made in reference to the Periodic Table of the Elements as published in Chemical and Engineering News, 63 (5) 27 1985. In this format, the groups are numbered 1-18. Catalysts based on metals and/or mixtures of metals from Groups 3, 4, 5, 6, 7, 8, 9, 10, 11, and 12 of the Periodic Table are effective. Different oxidations states are effective. The metal may be supported on a variety of inorganic and organic substrates and mixtures thereof including: pthalocyanine, aluminum oxide, and zinc oxide,

Preferred catalysts are based on the metals of Group 10. An example includes nickel.

After the alkane dehydrogenation step, water, carbon dioxide (if present), and oxygen (if present) are separated from the dehydrogenation product stream because such components can poison the polymerization catalyst. Additionally, water and carbon dioxide can be corrosive, and oxygen can cause decomposition, under high pressure.

Water is separated from the dehydrogenation product stream by methods known in the art, such as cryogenic distillation, adsorption, etc.

Typically, the dehydrogenation is carried out under conditions of high selectivity to the olefin. Oxidation to carbon dioxide is minimized. Carbon dioxide, if present, can be removed by conventional methods, e.g., cryogenic distillation, adsorption, and reaction.

Additional oxidative by-products, if present, are in small amounts. If by-products are present, separation can be effected by conventional methods such as distillation, adsorption, etc. Most or all of the unreacted alkane remains in the dehydrogenation product stream.

Oxygen, if present, is also separated from the dehydrogenation product stream. The separation can be carried out by methods known in the art such as cryogenic distillation, adsorption, etc.

The dehydrogenation product stream is then passed to a polymerization step where the alkene is contacted with a polymerization catalyst or initiator under reaction conditions to form a polymerization product stream comprising polyolefin, unreacted alkane, and optionally unreacted alkene. The polymerization step and catalyst or initiator can be any known in the art. Examples of processes useful for the polymerization step can be any one or combination of the high-pressure autoclave process, high-pressure tubular process, solution process, slurry-phase process, bulk-phase process, and/or gas-phase process discussed in Encyclopedia of Chemical Technology, 3rd Edition, 16, pp 385-470.

The process of the invention can be used to prepare LDPE, for example. In which case, the polymerization step can be carried out under conventional conditions using a free radical initator at high pressure (>20,000 psi) and temperature (>200°C). Other polymerizable comonomers may be present in the polymerization reactor. Examples of comonomers include vinyl acetate,methyl acrylate, and propylene.

The polymerization process can be conducted in the presence of at least one, or more, free radical initiators. As used herein, a free radical initiator is defined as a chemical substance that, under the polymerization conditions utilized, initiates chemical reactions by producing free radicals. Exemplary free radical initiators include organic peroxides such as tert-butyl peroxide; inorganic peroxides such as hydrogen peroxide-ferrous sulfate; azo compounds such as 2,2'-azobis[4-methoxy-2,4-dimethyl]pentanenitrile; carbon-carbon initiators such as 2,3-dimethyl-2,3-diphenylbutane; photo initiators such as benzophenone; and radiation, such as x-rays.

The free radical initiators are generally utilized in amounts of from about 1 to about 1000 ppm (parts per million), preferably from about 20 to about 300 ppm, and more preferably from about 50 to about 100 ppm, based on the total weight of the ethylene component of the polymer. Mixtures of free radical initiators can be used. The free radical initiators can be introduced into the polymerization process in any manner known in the art.

The process can also be used to prepare LLDPE or HDPE. In which case, the polymerization step can be carried out using conventional gas-phase, solution, or slurry polymerization conditions. Alternatively, the LLDPE and HDPE can be prepared at high pressure in an autoclave or tubular process. Catalysts for polymerization include Ziegler-Natta catalysts which typically contain a transition metal component and an organoaluminum component. Other catalysts include: chromium oxide catalysts; organochromium catalysts such as bis(triphenylsilyl) chromate supported on silica and activated with organoaluminum components; metallocene catalysts which typically consist of a transition metal having at least one substituted or unsubstituted cyclpentadienyl or cylcopentadienyl moiety and an organometallic component that is typically an alkyl aluminoxane or aryl substituted boron compound; single site catalysts as described in U. S. Patent 5,272,236; catalysts based on Groups 8, 9, 10 as described in U.S. Patent No. 5,866,663; Organometallics, 1998, 17, 3149-3151; and Journal of the American Chemical Society, 1998, 120, 7143-7144.

The above catalysts are or can be supported on an inert porous particulate carrier such as silicon dioxide and aluminum oxide.

The process can also be used to prepare polypropylene homopolymer, random copolymer, and block copolymer. Comonomers can be ethylene and/or higher α-olefins. In which case, the polymerization step can be carried out using conventional gas-phase, bulk-phase, or slurry polymerization conditions using a metallocene or Ziegler-Natta catalyst.

Exothermic heat from the dehydrogenation and polymerization steps can be recovered. For example, heat from the polymerization step may be recovered and used in the dehydrogenation step.

Following the polymerization step, the polyolefin formed can be separated from the unreacted alkane and the unreacted alkene (if present) using conventional techniques such as filtration, decantation, counter current stripping, degassing, and evaporation. The separated unreacted alkane and unreacted alkene may be recycled to the dehydrogenation step.

In one embodiment, the process according to the invention comprises the step of oligomerizing at least a portion of the alkene in the separated dehydrogenation product stream to form a mixture of α-olefins, such as 1-butene, 1-hexene, 1-octene, and 1-dodecene using conventional technology. Instead of conventional α-olefin separation and purification, the mixture of α-olefins can then be polymerized with the remaining portion of the alkene in the separated dehydrogenation product stream in the presence of a polymerization catalyst or initiator and the unreacted alkane to form a polymerization product stream comprising polyolefin and unreacted alkane.

This invention can be further illustrated by the following examples of preferred embodiments thereof, although it will be understood that these examples are included merely for purposes of illustration and are not intended to limit the scope of the invention.

### EXAMPLES

### Equipment Used to Assess Catalyst Performance

The bench unit reactor was a 2' x 1/4" OD stainless steel tubular reactor. The reactor was sheathed by a solid brass annulus 16" x 1" OD that was silver soldered to the stainless steel reactor. The brass annulus had a 1/16" hole drilled down to the outside surface of the stainless steel reactor corresponding to about 3/4 of the depth of the active catalyst bed that is contained in the stainless steel reactor tube. This hole contained a thermocouple used to control the reactor temperature.

The feed system to the reactor contained ethane and oxygen cylinders that had regulators that fed two different Brooks model 5850 Mass flow controllers that were calibrated for these two gases. There was a mixing manifold with about a 10' run of tubing before reaching the reactor inlet. The feed manifold also had a nitrogen flow controller for purging and shut down procedures.

The reactor was contained in an electrically heated vertical oven that was controlled by the thermocouple in the brass annulus (called the "skin temperature"). The exit gas was channeled to a multiple switching box for sampling and feeding the sample to an on-line Hewlett Packard HP6890 Gas Chromatographic automated system with a TC detector. The chromatograph was calibrated to record the mole percentage of oxygen and carbon dioxide. The mole percentages of ethane and product ethylene were calculated on a water-free basis from the chromatographic area counts. The reactor system was controlled by Camille Software.

### General Operating Procedure and Standard Flow Conditions

The catalysts to be evaluated in the reactor were ground to a powder in a mortar and pestle if the catalyst was not a powder as prepared. The catalyst charge contained 1.00 ml of powdered catalyst mixed with 2.00 ml of 50-70 mesh silica sea sand diluent. The two components were weighed into a beaker based on their bulk densities to get accurate volumes for the charge. These were mixed mechanically. The bed was loaded manually in the following sequence from the bottom exit to the top:
0.20 grams of glass wool
1.0 ml of 20-25 mesh Denstone packing
0.20 grams of glass wool
3.00 ml of the catalyst / sand mixture
0.20 grams of glass wool

The runs were initiated by starting the ethane at a standard gas feed rate of 120 cc/min STP and oxygen at 6 cc/min STP. The reactor was then heated to the target reactor (skin) temperature. The temperature was allowed to equilibrate for thirty minutes before recording data and shooting the on-line gas chromatogram of the product off-gas. Typical runs varied the temperature and reactant flows to obtain data at other points. The reactor was allowed to equilibrate normally for thirty minutes before taking data and shooting the on-line gas chromatogram of the reactor off-gas.

### Example 1

### Ethane Oxidative Dehydrogenation to Ethylene Using Commercial Nickel Hydrogenation Catalyst Kataleuna KL 6515 TL(1.2)

The commercial 1/16" extrudate form of the title catalyst was ground to powder in a mortar and pestle. A mix of the powdered catalyst (1.09 gm = 1.00 ml) and 50-70 mesh silica sea sand (3.20 gm = 2.00 ml) were mixed together for the active catalyst part of the reactor charge. The reactor was loaded as recorded earlier. Table 1 below summarizes the results.

**Table 1**

| Temp. (°C) | C₂H₆ (sccm) | O₂ (sccm) | % Conversion O₂ | % Selectivity to C₂H₄ on Consumed Ethane | Mole % C₂H₄ in Product |
|---|---|---|---|---|---|
| 250 | 120 | 6 | 31 | 60 | 0.56 |
| 275 | 120 | 6 | 59 | 61 | 1.19 |
| 300 | 120 | 6 | 89 | 62 | 2.00 |
| 300 | 120 | 3 | 100 | 72 | 1.59 |
| 300 | 86 | 5 | 83 | 68 | 1.18 |
| 300 | 120 | 9 | 76 | 51 | 1.67 |
| 325 | 120 | 6 | 100 | 64 | 2.48 |

### Example 2

### Ethane Oxidative Dehydrogenation to Ethylene Using Commercial Nickel Hydrogenation Catalyst Engelhard Ni-3314

The commercial catalyst extrudates were ground to powder in a mortar and pestle. A mix of the powder catalyst (0.99 gm = 1.00 ml) and 50-70 mesh silica sea sand (3.20 gm = 2.00 ml) were mixed together for the active catalyst part of the reactor charge. The reactor was loaded as recorded earlier. Table 2 below summarizes the results.

**Table 2**

| Temp. (°C) | C₂H₆ (sccm) | O₂ (sccm) | % Conversion O₂ | % Selectivity to C₂H₄ on Consumed Ethane | Mole % C₂H₄ in Product |
|---|---|---|---|---|---|
| 250 | 120 | 6 | 25 | 57 | 0.39 |
| 275 | 120 | 6 | 48 | 56 | 0.80 |
| 300 | 120 | 6 | 79 | 57 | 1.50 |
| 300 | 120 | 3 | 100 | 66 | 1.22 |
| 300 | 120 | 9 | 68 | 46 | 1.23 |
| 300 | 80 | 6 | 75 | 48 | 1.47 |
| 325 | 120 | 6 | 100 | 58 | 1.98 |

### Example 3

### Ethane Oxidative Dehydrogenation Run Using Commercial Engelhard Ni-3314 Hydrogenation Catalyst Modified with 1 Weight % Copper

The catalyst was prepared by the following method:

Anhydrous copper sulfate (125.6 mg having 50 mg of copper as metal) was dissolved in 100 ml of de-ionized water in a 500 ml Erlenmeyer flask. Powdered N-3314 catalyst (5.00 gm) was added to the stirred mixture at ambient conditions. This was heated with stirring to 60 degrees Celsius. A solution of 160 mg of sodium formate in 10 ml of de-ionized water was prepared separately. The sodium formate was added to the hot stirred mixture at 60 degrees Celsius over two minutes. The mixture was stirred an additional 15 minutes at 60 degrees and then cooled to room temperature. The black solid catalyst powder was filtered on polyamide filter paper and washed with 50 ml of de-ionized water. The moist powder paste was dried at room temperature with a stream of nitrogen. The net weight of recovered catalyst was 5.06 grams.

The above catalyst (0.85 grams = 1.00 ml) and 50-70 mesh silica sea sand (3.20 grams = 2.00 ml) were mixed together and charged to the reactor as described earlier. Table 3 below summarizes the results of the run.

**Table 3**

| Temp. (°C) | C₂H₆ (sccm) | O₂ (sccm) | % Conversion O₂ | % Selectivity to C₂H₄ on Consumed Ethane | Mole % C₂H₄ in Product |
|---|---|---|---|---|---|
| 250 | 120 | 6 | 11 | 52 | 0.16 |
| 275 | 120 | 6 | 25 | 51 | 0.36 |
| 300 | 120 | 6 | 46 | 53 | 0.70 |
| 325 | 120 | 6 | 68 | 55 | 1.17 |
| 350 | 120 | 6 | 92 | 60 | 1.92 |
| 350 | 120 | 3 | 100 | 69 | 1.45 |
| 350 | 120 | 9 | 82 | 49 | 1.74 |

### Example 4

### Ethane Oxidative Dehydrogenation Run Using Commercial Engelhard Ni-3314 Hydrogenation Catalyst Modified with 1 Weight % Bismuth

The catalyst was prepared by the following method:

Bismuth (III) nitrate pentahydrate (116.1 mg containing 50 mg of Bi as metal) was dispersed into 100 ml of de-ionized water in a 500 ml Erlenmeyer flask at ambient temperature. Powdered Ni-3314 catalyst was added to the stirred solution at ambient temperature. This was heated to 60 degrees Celsius and kept at 60 degrees for 30 minutes. This was cooled to ambient temperature and filtered on a polyamide filter paper. The filter cake was washed with 50 ml of de-ionized water. The moist powder paste was dried at ambient temperature with a stream of nitrogen. Net wt 5.36 grams.

The above catalyst (0.96 grams = 1.00 ml) and 50-70 mesh of sea sand (3.20 grams = 2.00 ml) were mixed and charged to the reactor as described previously. Table 4 below shows the results of the run.

**Table 4**

| Temp. (°C) | C₂H₆ (sccm) | O₂ (sccm) | % Conversion O₂ | % Selectivity to C₂H₄ on Consumed Ethane | Mole % C₂H₄ in Product |
|---|---|---|---|---|---|
| 275 | 120 | 6 | 33 | 34 | 0.27 |
| 300 | 120 | 6 | 58 | 39 | 0.58 |
| 325 | 120 | 6 | 81 | 46 | 1.09 |
| 325 | 120 | 3 | 85 | 63 | 1.05 |
| 325 | 120 | 9 | 73 | 31 | 0.80 |
| 325 | 120 | 6 | 75 | 40 | 0.82 |
| 350 | 120 | 6 | 92 | 52 | 1.54 |
| 350 | 120 | 3 | 100 | 66 | 1.30 |

### Example 5

### Ethane Oxidative Dehydrogenation Run Using 6.92 Weight % Nickel Encapsulated in 13 X Zeolite

20.0 grams of 13 X Mole Sieve Zeolite (Aldrich) extrudates was added to a 500 ml Erlenmeyer flask having a magnetic stir bar along with 50 ml of de-ionized water. Nickel (II) formate dehydrate powder (6.00 grams containing 1.91 grams of nickel as metal) was added to the slurried mole sieves. This mixture was stirred at ambient conditions for 72 hours. During this time the 1/16 extrudates of the Mole Sieves disintegrated into a slurried powder. The slurry was filtered on #5 Whatman filter paper and washed with 50 ml of de-ionized water. The pale green filtrate (volume 94 ml) had a nickel content of 1502 mg Ni/liter for a total of 0.141 grams of contained nickel. The amount of nickel contained in the 13 X zeolite was calculated to be 1.77 grams. The solid was dried at 80 degrees for five days. Net wt of pale green powder 25.53 grams. The nickel is presumed to be contained in the pores as the formate.

Nickel formate absorbed 13 X zeolite (produced in the previous paragraph description) (0.69 grams = 1.00 ml) and 50-70 mesh silica sea sand (3.20 gram = 2.00 ml) were mixed and charged to the reactor as described earlier. The catalyst was activated in-situ by heating the reactor to 180-200 degrees Celsius for 30 minutes with a flow of 120 sccm of ethane and no oxygen. The reactor off-gas was analyzed chromatographically and the presence of formate decomposition products carbon monoxide and carbon dioxide were observed. The active catalyst is now presumed to be encapsulated clusters of nickel metal and nickel metal hydride complex. Oxygen feed was started at 6 sccm for the initial start of the run at the first temperature setting of 300 degrees Celsius. Table 5 shows the results of the run.

**Table 5**

| Temp. (°C) | C₂H₆ (sccm) | O₂ (sccm) | % Conversion O₂ | % Selectivity to C₂H₄ on Consumed Ethane | Mole % C₂H₄ in Product |
|---|---|---|---|---|---|
| 300 | 120 | 6 | 7 | N/D | 0.13 |
| 350 | 120 | 6 | 26 | 57 | 0.46 |
| 400 | 120 | 6 | 44 | 55 | 0.73 |
| 450 | 120 | 6 | 70 | 55 | 1.24 |
| 450 | 120 | 3 | 76 | 61 | 0.87 |
| 450 | 120 | 9 | 57 | 47 | 1.07 |

The above examples demonstrate that ethane can be oxidized to ethylene using commercially available nickel-based hydrogenation catalysts. A new composition for this catalytic application, namely encapsulated nickel in 13 X zeolite has also been demonstrated to be an effective catalyst for the conversion of ethane into ethylene by oxidative dehydrogenation.

### Example 6

### Preparation of LLDPE

Ethane is partially and selectively dehydrogenated with oxygen to give ethylene and water.

Water is separated from ethylene and ethane by cryogenic distillation. No other separation is performed.

A partial stream of ethylene in the presence of ethane is oligomerized to form 1-butene, 1-hexene, 1-octene, and 1-dodecene. No additional separation is performed

The mixture of the remainder of ethylene and ethane from the dehydrogenation reaction and the mixture from the oligomerization reaction are fed to a gas-phase polymerization reactor with Ziegler-Natta catalyst to make LLDPE.

Solid polyethylene is separated from unreacted ethane, which is recycled to the dehydrogenation reaction.

### Example 7

### Preparation of LLDPE Using Purified Alpha-olefins

Ethane is partially and selectively dehydrogenated with oxygen to give ethylene and water.

Water is separated from ethylene and ethane by adsorption beds. No other separation is performed.

The mixture of the remainder of ethylene and ethane from the dehydrogenation reaction and purified α-olefin (1-butene, 1-hexene, or 1-octene) are fed to a gas-phase polymerization reactor with a Ziegler-Natta catalyst to make polyethylene.

Solid LLDPE is separated from unreacted ethane, which is recycled to the dehydrogenation reaction.

### Example 8

### Preparation of LDPE

Ethane is partially and selectively dehydrogenated with oxygen to give ethylene and water.

Water is separated from ethylene and ethane by cryogenic distillation. No other separation is performed.

The mixture of ethylene and ethane from the dehydrogenation reaction is fed to a high-pressure reactor with a peroxide initiator to make LDPE.

Solid LDPE is separated from unreacted ethane, which is recycled to the dehydrogenation reaction.

### Example 9

### Preparation of Polypropylene Homopolymer (PP)

Propane is partially and selectively dehydrogenated with oxygen to give propylene and water.

Water is separated from propylene and propane by cryogenic distillation. No other separation is performed.

The mixture of the propylene and propane from the dehydrogenation reaction is fed to a gas-phase reactor with a Ziegler-Natta catalyst to make PP.

Solid polypropylene is separated from unreacted propane, which is recycled to the dehydrogenation reaction.

### Example 10

### Preparation of Propylene-Ethylene Copolymer (P-Et)

Propane is partially and selectively dehydrogenated with oxygen to give propylene and water.

Water is separated from propylene and propane by adsorption beds. No other separation is performed.

The mixture of propylene and propane from the dehydrogenation reaction is fed to a gas-phase reactor with ethylene and a Ziegler Natta catalyst to make P-Et.

Solid polypropylene-ethylene copolymer is separated from unreacted propane, which is recycled to the dehydrogenation reaction.

The invention has been described in detail with particular reference to preferred embodiments thereof, but it will be understood that variations and modifications can be effected within the spirit and scope of the invention.

## Claims

1. A process for making polyolefin from an alkane, comprising:
(a) dehydrogenating an alkane in the presence of oxygen to form a dehydrogenation product stream comprising a corresponding alkene, unreacted alkane, and water, and optionally other by-products and oxygen;
(b) separating the water, other by-products (if present), and oxygen (if present) from the dehydrogenation product stream without separating the unreacted alkane to form a separated dehydrogenation product stream comprising alkene and unreacted alkane;
(c) polymerizing the alkene in the separated dehydrogenation product stream in the presence of a polymerization catalyst or initiator and the unreacted alkane to form a polymerization product stream comprising polyolefin, unreacted alkane, and optionally unreacted alkene;
(d) separating the polyolefin from the unreacted alkane and unreacted alkene (if present) in the polymerization product stream; and
(f) recycling of the unreacted alkane and unreacted alkene (if present) to the dehydrogenation step.

2. The process according to claim 1, wherein the polyolefin is polyethylene and the alkane is ethane.

3. The process according to claim 2, wherein the polyethylene is low density polyethylene, linear low density polyethylene, or high density polyethylene.

4. The process according to claim 2, wherein the polymerization step is carried out under high pressure in an autoclave or tubular reactor, or in solution, a slurry, or gas phase, or combinations thereof.

5. The process according to claim 2, wherein the polymerization catalyst is a Ziegler-Natta, metallocene, single site, late transition metal, or chromium catalyst, or combinations thereof.

6. The process according to claim 1, wherein the polyolefin is an ethylene copolymer.

7. The process according to claim 1, wherein the polyolefin is polypropylene, and the alkane is propane.

8. The process according to claim 7, wherein the polymerization step is carried out in a slurry, in bulk, or in the gas phase, or combinations thereof.

9. The process according to claim 7, wherein the polypropylene is a homopolymer.

10. The process according to claim 1, wherein the polymerization step is carried out in the presence of an α-olefin.

11. The process according to claim 10, wherein the α-olefin is 1-butene, 1-hexene, 1-octene, 1-dodecene, or combinations thereof.

12. The process according to claim 1, further comprising oligomerizing at least a portion of the alkene in the separated dehydrogenation product stream to form a mixture of α-olefins, and polymerizing the alkene in the unoligomerized portion of the separated dehydrogenation product stream with the mixture of α-olefins in the presence of a polymerization catalyst or initiator and the unreacted alkane to form a polymerization product stream comprising polyolefin and unreacted alkane.

13. The process according to claim 1, further comprising recovering heat liberated from the dehydrogenation step.

14. The process according to claim 13, wherein the heat recovered from the polymerization step is used in the dehydrogenation step.

15. The process according to claim 7, wherein the polymerization catalyst is a Ziegler-Natta, metallocene, or single site catalyst, or combinations thereof.

## Patentansprüche

1. Verfahren zur Herstellung von Polyolefin aus einem Alkan, umfassend:
(a) Dehydrieren von einem Alkan in Gegenwart von Sauerstoff, um einen Dehydrierungs-Produkt-Strom, umfassend ein entsprechendes Alken, nicht umgesetztes Alkan und Wasser und gegebenenfalls andere Nebenprodukte und Sauerstoff, zu bilden;
(b) Abtrennen von dem Wasser, anderen Nebenprodukten (falls vorliegend) und Sauerstoff (falls vorliegend) aus dem Dehydrierungs-Produkt-Strom ohne Abtrennen des nicht umgesetzten Alkans, um einen getrennten Dehydrierungs-Produkt-Strom, umfassend Alken und nicht umgesetztes Alkan, zu bilden;
(c) Polymerisieren des Alkens in dem getrennten Dehydrierungs-Produkt-Strom in Gegenwart von einem Polymerisations-Katalysator oder -Starter und dem nicht umgesetzten Alkan, um einen Polymerisations-Produkt-Strom, umfassend Polyolefin, nicht umgesetztes Alkan und gegebenenfalls nicht umgesetztes Alken, zu bilden;
(d) Abtrennen des Polyolefins aus dem nicht umgesetzten Alkan und nicht umgesetzten Alken (falls vorliegend) in dem Polymerisations-Produkt-Strom; und
(f) Zurück-Führen von dem nicht umgesetzten Alkan und nicht umgesetzten Alken (falls vorliegend) zu dem Dehydrierungs-Schritt.

2. Verfahren nach Anspruch 1, wobei das Polyolefin Polyethylen ist und das Alkan Ethan ist.

3. Verfahren nach Anspruch 2, wobei das Polyethylen niederdichtes Polyethylen bzw. Hochdruck-Polyethylen, linear niederdichtes Polyethylen bzw. lineares Hochdruck-Polyethylen oder hochdichtes Polyethylen bzw. Niederdruck-Polyethylen ist.

4. Verfahren nach Anspruch 2, wobei der Polymerisations-Schritt unter hohem Druck in einem Autoklaven- oder Röhren-Reaktor oder in Lösung, einer Aufschlämmung oder Gas-Phase oder Kombinationen davon ausgeführt wird.

5. Verfahren nach Anspruch 2, wobei der Polymerisations-Katalysator ein Ziegler-Natta-, Metallocen-, Single-Site-, später Übergangs-Metall- oder Chrom-Katalysator oder Kombinationen davon ist.

6. Verfahren nach Anspruch 1, wobei das Polyolefin ein EthylenCopolymer ist.

7. Verfahren nach Anspruch 1, wobei das Polyolefin Polypropylen ist und das Alkan Propan ist.

8. Verfahren nach Anspruch 7, wobei der Polymerisations-Schritt in einer Aufschlämmung, in der Masse oder in der Gas-Phase oder Kombinationen davon ausgeführt wird.

9. Verfahren nach Anspruch 7, wobei das Polypropylen ein Homopolymer ist.

10. Verfahren nach Anspruch 1, wobei der Polymerisations-Schritt in Gegenwart von einem α-Olefin ausgeführt wird.

11. Verfahren nach Anspruch 10, wobei das α-Olefin 1-Buten, 1-Hexen, 1-Octen, 1-Dodecen oder Kombinationen davon ist.

12. Verfahren nach Anspruch 1, weiterhin umfassend Oligomerisieren von mindestens einem Teil von dem Alken in dem getrennten Dehydrierungs-Produkt-Strom, um ein Gemisch von α-Olefinen zu bilden, und Polymerisieren des Alkens in dem nicht oligomerisierten Teil von dem getrennten Dehydrierungs-Produkt-Strom mit dem Gemisch von α-Olefinen in Gegenwart von einem Polymerisations-Katalysator oder -Starter und dem nicht umgesetzten Alkan, um einen Polymerisations-Produkt-Strom, umfassend Polyolefin und nicht umgesetztes Alkan, zu bilden.

13. Verfahren nach Anspruch 1, weiterhin umfassend WiederGewinnen von aus dem Dehydrierungs-Schritt freigesetzter Wärme.

14. Verfahren nach Anspruch 13, wobei die aus dem Polymerisations-Schritt wiedergewonnene Wärme in dem Dehydrierungs-Schritt verwendet wird.

15. Verfahren nach Anspruch 7, wobei der Polymerisations-Katalysator ein Ziegler-Natta-, Metallocen- oder Single-Site-Katalysator oder Kombinationen davon ist.

## Revendications

1. Procédé pour la préparation d'une polyoléfine à partir d'un d'alcane, comprenant le fait de :
(a) déshydrogéner un alcane en présence d'oxygène pour obtenir un courant de produits de déshydrogénation qui comprend un alcène correspondant, l'alcane n'ayant pas réagi et de l'eau, et de manière facultative d'autres sous-produits, et de l'oxygène ;
(b) séparer l'eau, les autres sous-produits (lorsqu'ils sont présents) et l'oxygène (lorsqu'il est présent) du courant de produits de déshydrogénation, sans séparer l'alcane n'ayant pas réagi, pour obtenir un courant de produits de déshydrogénation séparé comprenant un alcène et l'alcane n'ayant pas réagi ;
(c) polymériser l'alcène dans le courant de produits de déshydrogénation séparé en présence d'un catalyseur ou d'un initiateur de la polymérisation et l'alcane n'ayant pas réagi pour obtenir un courant de produits de polymérisation comprenant de la polyoléfine, l'alcane n'ayant pas réagi et, de manière facultative, l'alcène n'ayant pas réagi ;
(d) séparer la polyoléfine de l'alcane n'ayant pas réagi et de l'alcène n'ayant pas réagi (lorsqu'il est présent) dans le courant de produits de polymérisation ; et
(e) recycler l'alcane n'ayant pas réagi et l'alcène n'ayant pas réagi (lorsqu'il est présent) dans l'étape de déshydrogénation.

2. Procédé selon la revendication 1, dans lequel la polyoléfine est du polyéthylène et l'alcane est de l'éthane.

3. Procédé selon la revendication 2, dans lequel le polyéthylène est du polyéthylène basse densité, du polyéthylène linéaire basse densité ou du polyéthylène haute densité.

4. Procédé selon la revendication 2, dans lequel l'étape de polymérisation est mise en oeuvre sous pression élevée dans un autoclave ou dans un réacteur tubulaire, ou bien en solution, en suspension ou en phase gazeuse, ou bien dans leurs combinaisons.

5. Procédé selon la revendication 2, dans lequel le catalyseur de polymérisation est un catalyseur de type Ziegler-Natta, un catalyseur de métallocène, un catalyseur à site unique, un catalyseur à base d'un métal de transition tardive ou un catalyseur à base de chrome, ou leurs combinaisons.

6. Procédé selon la revendication 1, dans lequel la polyoléfine est un copolymère d'éthylène.

7. Procédé selon la revendication 1, dans lequel la polyoléfine est du polypropylène et l'alcane est du propane.

8. Procédé selon la revendication 7, dans lequel l'étape de polymérisation est mise en oeuvre en suspension, en masse ou en phase gazeuse, ou dans leurs combinaisons.

9. Procédé selon la revendication 7, dans lequel le polypropylène est un homopolymère.

10. Procédé selon la revendication 1, dans lequel l'étape de polymérisation est mise en oeuvre en présence d'une α-oléfine.

11. Procédé selon la revendication 10, dans lequel l'α-oléfine est le 1-butène, le 1-hexène, le 1-octène, le 1-dodécène ou leurs combinaisons.

12. Procédé selon la revendication 1, comprenant en outre l'oligomérisation d'au moins une portion de l'alcène dans le courant de produits de déshydrogénation séparé, pour obtenir un mélange d'α-oléfines, et la polymérisation de l'alcène dans la portion non oligomérisée du courant de produits de déshydrogénation séparé avec le mélange d'α-oléfines en présence d'un catalyseur ou d'un initiateur de la polymérisation et l'alcane n'ayant pas réagi pour obtenir un courant de produits de polymérisation comprenant de la polyoléfine et l'alcane n'ayant pas réagi.

13. Procédé selon la revendication 1, comprenant en outre la récupération de la chaleur libérée par l'étape de déshydrogénation.

14. Procédé selon la revendication 13, dans lequel la chaleur récupérée de l'étape de polymérisation est utilisée dans l'étape de déshydrogénation.

15. Procédé selon la revendication 7, dans lequel le catalyseur de la polymérisation est un catalyseur de type Ziegler-Natta, un catalyseur de métallocène ou un catalyseur à site unique, ou leurs combinaisons.
